# EUROPEAN PATENT SPECIFICATION

(11) **EP 1 499 590 B1**
(45) Date of publication and mention of the grant of the patent: **03.05.2006**
(21) Application number: 02793633.5
(22) Date of filing: 11.12.2002
(51) Int. Cl.: C07D 211/58, A61K 31/4468

(54) **Piperidine derivatives as potassium channel blockers**
Piperidinderivate als Kalium-Kanal Blockers
Dérivés de pipéridine comme agents bloquants du canal potassium

(30) Priority: 14.12.2001 SE 0104251
(43) Date of publication of application: 26.01.2005
(73) Proprietor: AstraZeneca AB, 151 85 Södertälje (SE)
(72) Inventor: ERIKSSON, Tomas, S-221 87 Lund (SE); NOORI, Ghazi, S-224 71 Lund (SE)
(86) International application number: PCT/SE2002/002304
(87) International publication number: WO 2003/051839

(56) References cited:
- WO-A1-01/62728
- WO-A1-01/62757

## Description

The present invention relates to novel compounds, processes for their preparation, pharmaceutical compositions containing them and their use in therapy.

Chemokines play an important role in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C) and Cys-Cys (C-C) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α, and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

International patent application no. PCT/SE01/01377 describes compounds, having MIP-1α chemokine receptor activity, that are of formula 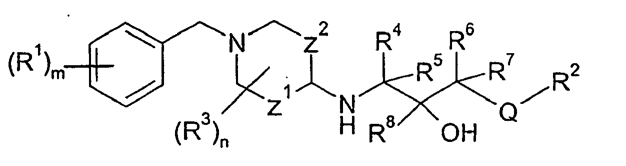 wherein
m is 0, 1, 2 or 3;
each R¹ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR⁹R¹⁰, C₃-C₆ cycloalkylamino, C₁-C₆ alkylthio, C₁-C₆ alkylcarbonyl, C₁-C₆ alkylcarbonylamino, sulphonamido, C₁-C₆ allcylsulphonyl, -C(O)NR¹¹R¹², -NR¹³C(O)-(NH)ₚR¹⁴, phenyl, or C₁-C₆ alkyl optionally substituted by carboxyl or C₁-C₆ alkoxycarbonyl;
p is 0 or 1;
Z¹ represents a bond or a group (CH₂)_{q} where q is 1 or 2;
Z² represents a bond or a group CH₂, with the proviso that Z¹ and Z² do not both simultaneously represent a bond;
Q represents an oxygen or sulphur atom or a group CH₂ or NH;
R² represents a group 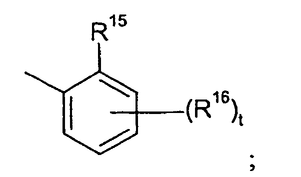 n is 0, 1 or 2;
each R³ independently represents a C₁-C₆ alkyl, C₁-C₆ alkoxycarbonyl, -CH₂OH or carboxyl group;
R⁴, R⁵, R⁶ and R each independently represent a hydrogen atom or a C₁-C₆ alkyl group, or R⁴, R⁵, R⁶ and R⁷ together represent a C₁-C₄ alkylene chain linking the two carbon atoms to which they are attached to form a 4- to 7-membered saturated carbocycle, or R⁵, R⁶ and R⁷ each represent a hydrogen atom and R⁴ and R⁸ together with the carbon atoms to which they are attached form a 5- to 6-membered saturated carbocycle;
R⁸ represents a hydrogen atom, a C₁-C₆ alkyl group or is linked to R⁴ as defined above;
R⁹ and R¹⁰ each independently represent a hydrogen atom or a C₁-C₆ alkyl group, or R⁹ and R¹⁰ together with the nitrogen atom to which they are attached form a 4- to 7- membered saturated heterocycle;
R¹¹ and R¹² each independently represent a hydrogen atom or a C₁-C₆ alkyl group optionally substituted by C₁-C₆ alkoxycarbonyl;
R¹³ represents a hydrogen atom or a C₁-C₆ alkyl group;
R¹⁴ represents a hydrogen atom, or a C₁-C₆ alkyl group optionally substituted by carboxyl, C₁-C₆ alkoxy or C₁-C₆ alkoxycarbonyl;
R¹⁵ represents carboxyl, C₁-C₆ alkoxy, C₁-C₆ alkylcarbonyl, C₁-C₆ alkoxycarbonyl, C₁-C₆ alkoxycarbonylC₁-C₆ alkyl or a group -NR¹⁷ R¹⁸, -NHSO₂CH₃, -C(O)NR¹⁷R¹⁸, -NHC(O)NR¹⁷R¹⁸, -OC(O)NR¹⁷R¹⁸, -OCH₂C(O)NR¹⁷R¹⁸, -NHC(O)OR¹⁹ or -NHC(O)R²⁰;
t is 0, 1, 2 or 3;
each R¹⁶ independently represents halogen, cyano, nitro, carboxyl, hydroxyl, C₃-C₆ cycloalkyl, C₁-C₆ alkoxy, C₁-C₆ alkoxycarbonyl, C₁-C₆ haloalkyl, C₁-C₆ haloalkoxy, -NR²¹R²², C₃-C₆ cycloalkylamino, C₁-C₆ alkylthio, C₁-C₆ alkylcarbonyl, C₁-C₆ alkylcarbonylamino, sulphonamido, C₁-C₆ alkylsulphonyl, -C(O)NR²³R²⁴, -NR²⁵C(O)(NH)ᵥR²⁶, phenyl, or C₁-C₆ alkyl optionally substituted by carboxyl or C₁-C₆ alkoxycarbonyl;
R¹⁷ and R¹⁸ each independently represent a hydrogen atom, or a C₁-C₆ alkyl group optionally substituted by carboxyl or C₁-C₆ alkoxycarbonyl, or R¹⁷ and R¹⁸ together with the nitrogen atom to which they are attached form a 4- to 7-membered saturated heterocycle;
R¹⁹ represents a hydrogen atom, or a C₁-C₆ alkyl group optionally substituted by carboxyl or C₁-C₆ alkoxycarbonyl;
R²⁰ represents a group C₁-C₆ alkyl, C₂-C₆ alkenyl, C₃-C₆ cycloalkyl, adamantyl, C₅-C₆ cycloalkenyl, phenyl or a saturated or unsaturated 5- to 10-membered heterocyclic ring system comprising at least one heteroatom selected from nitrogen, oxygen and sulphur, each of which may be optionally substituted by one or more substituents independently selected from nitro, hydroxyl, oxo, halogen, carboxyl, C₁-C₆ alkyl, C₁-C₆ alkoxy, C₁-C₆ alkylthio, C₁-C₆ alkylcarbonyl, C₁-C₆ alkoxycarbonyl, phenyl and -NHC(O)-R²⁷;
R²¹ and R²² each independently represent a hydrogen atom or a C₁-C₆ alkyl group, or R²¹ and R²² together with the nitrogen atom to which they are attached form a 4- to 7- membered saturated heterocycle;
R²³ and R²⁴ each independently represent a hydrogen atom or a C₁-C₆ alkyl group optionally substituted by C₁-C₆ alkoxycarbonyl;
vis 0 or 1;
R²⁵ represents a hydrogen atom or a C₁-C₆ alkyl group;
R²⁶ represents a hydrogen atom, or a C₁-C₆ alkyl group optionally substituted by carboxyl, C₁-C₆ alkoxy or C₁-C₆ alkoxycarbonyl; and
R²⁷ represents a C₁-C₆ alkyl, amino or phenyl group;
and pharmaceutically acceptable salts and solvates thereof.

Studies of the metabolic pathways in which compounds of International patent application no. PCT/SE01/01377 are degraded reveal the formation of several different metabolites. In one such pathway, compound (drug) reacts with glucoronic acid to form glucoronides. Glucoronidation increases the drug's water solubility and thus facilitates renal excretion of the drug. It is preferred if the major metabolite is glucoronidated.

It is now surprisingly been found that certain compounds within the general teaching of International patent application no. PCT/SE01/01377 have, inter alia, advantageous stability properties *in vivo* leading to their increased metabolism via the glucoronidation pathway.

In accordance with the present invention, there is therefore provided a compound of formula 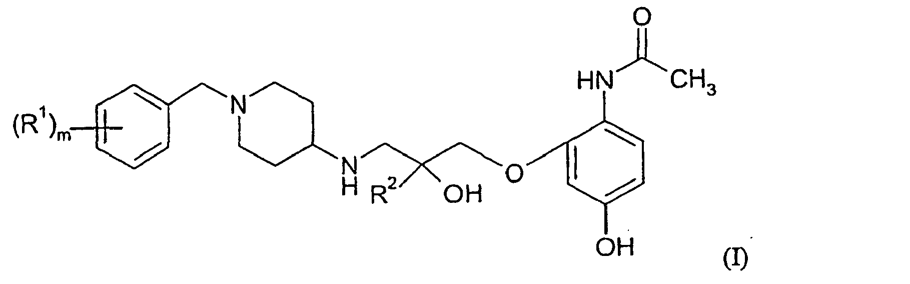 wherein
m is 0, 1 or 2;
each R¹ independently represents halogen or cyano; and
R² represents a hydrogen atom or methyl group;
or a pharmaceutically acceptable salt or solvate thereof.

The compounds of the invention have also been found to act as potassium channel blockers and are positive in a hERG assay; it should be noted that such activity may give rise to ECG (electrocardiogram) changes *in vivo.*

Without being bound to any particular theory, it is believed that the advantageous properties of the compounds of the invention are at least due in part to the presence of, and point of attachment of, the hydroxyl substituent group on the benzene ring on the righthand side of the molecule in formula (I).

In one embodiment of the invention, m is 1 and R¹ represents a halogen atom such as a fluorine, chlorine, bromine or iodine atom, particularly a chlorine atom, e.g. in the 4-position of the benzene ring relative to the carbon atom to which the CH₂ linking group binds.

In another embodiment of the invention, R² represents a methyl group.

In a further embodiment of the invention, the compound is 4-({(2S)-3-[2-(acetylamino)-5-hydroxyphenoxy]-2-hydroxy-2-methylpropyl}ammonio)-1-(4-chlorobenzyl)piperidine or a pharmaceutically acceptable salt or solvate thereof.

The present invention further provides a process for the preparation of a compound of formula (I) or a pharmaceutically acceptable salt or solvate thereof as defined above which comprises
(a) reacting a compound of formula 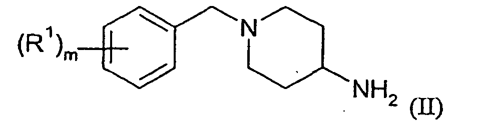 wherein m and R¹ are as defined in formula (I), with a compound of formula 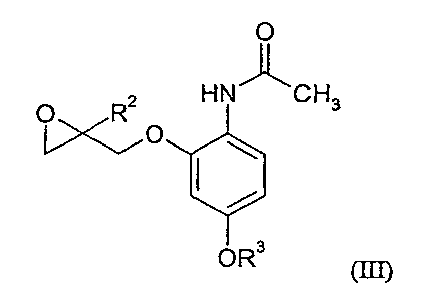 wherein R³ represents a hydrogen atom or a suitable protecting group and R² is as defined in formula (I); or
(b) reacting a compound of formula 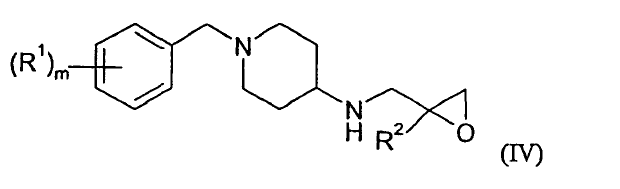 wherein m, R¹ and R² are as defined in formula (I), with a compound of formula 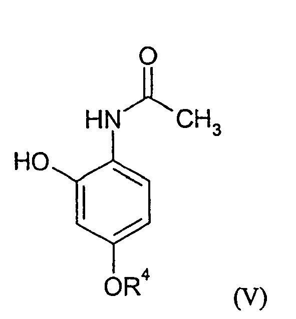 wherein R⁴ represents a hydrogen atom or a suitable protecting group; or
(c) reacting a compound of formula 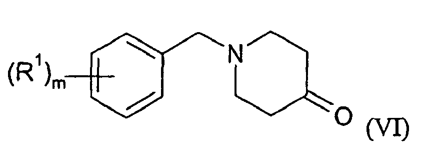 wherein m and R¹ are as defined in formula (I), with a compound of formula 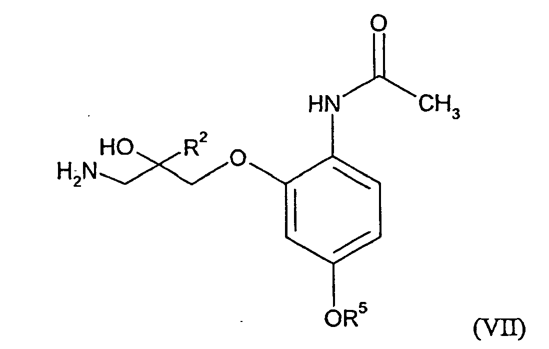 wherein R⁵ represents a hydrogen atom or a suitable protecting group and R² is as defined in formula (I);
   and optionally after (a), (b) or (c) forming a pharmaceutically acceptable salt or solvate of the compound of formula (I).

The process of the invention may conveniently be carried out in a solvent, e.g. an organic solvent such as an alcohol (e.g. methanol or ethanol), a hydrocarbon (e.g. toluene) or acetonitrile at a temperature of, for example, 15°C or above such as a temperature in the range from 20 to 120°C.

Compounds of formulae (II), (III), (IV), (V), (VI) and (VII) are either commercially available, are well known in the literature or may be prepared easily using known techniques.

It will be appreciated by those skilled in the art that in the process of the present invention certain functional groups such as hydroxyl or amino groups in the starting reagents or intermediate compounds may need to be protected by protecting groups. Thus, the preparation of the compounds of formula (I) may involve, at an appropriate stage, the removal of one or more protecting groups.

The protection and deprotection of functional groups is described in 'Protective Groups in Organic Chemistry', edited by J.W.F. McOmie, Plenum Press (1973) and Protective . Groups in Organic Synthesis', 2nd edition, T.W. Greene and P.G.M. Wuts, Wiley-Interscience (1991).

The compounds of formula (I) above may be converted to a pharmaceutically acceptable salt or solvate thereof, preferably an acid addition salt such as a hydrochloride, trifluoroacetate (e.g. ditrifluoroacetate), hydrobromide, phosphate, acetate, fumarate, maleate, tartrate, citrate, oxalate, methanesulphonate or *p*-toluenesulphonate.

Compounds of formula (I) are capable of existing in stereoisomeric forms. It will be understood that the invention encompasses the use of all geometric and optical isomers (including atropisomers) of the compounds of formula (I) and mixtures thereof including racemates. The use of tautomers and mixtures thereof also form an aspect of the present invention. Preferred optical isomers are the (S)-enantiomers.

The compounds of formula (I) have activity as pharmaceuticals, in particular as modulators of chemokine receptor (especially MIP-1α chemokine receptor) activity, and may be used in the treatment of autoimmune, inflammatory, proliferative and hyperproliferative diseases and immunologically-mediated diseases including rejection of transplanted organs or tissues and Acquired Immunodeficiency Syndrome (AIDS).

Examples of these conditions are:
(1) (**the respiratory tract**) airways diseases including chronic obstructive pulmonary disease (COPD) such as irreversible COPD; asthma, such as bronchial, allergic, intrinsic, extrinsic and dust asthma, particularly chronic or inveterate asthma (e.g. late asthma and airways hyper-responsiveness); bronchitis; acute, allergic, atrophic rhinitis and chronic rhinitis including rhinitis caseosa, hypertrophic rhinitis, rhinitis purulenta, rhinitis sicca and rhinitis medicamentosa; membranous rhinitis including croupous, fibrinous and pseudomembranous rhinitis and scrofoulous rhinitis; seasonal rhinitis including rhinitis nervosa (hay fever) and vasomotor rhinitis; sarcoidosis, farmer's lung and related diseases, fibroid lung and idiopathic interstitial pneumonia;
(2) (**bone and joints**) rheumatoid arthritis, seronegative spondyloarthropathies (including ankylosing spondylitis, psoriatic arthritis and Reiter's disease), Behcet's disease, Sjogren's syndrome and systemic sclerosis;
(3) (**skin**) psoriasis, atopical dermatitis, contact dermatitis and other eczmatous dermitides, seborrhoetic dermatitis, Lichen planus, Pemphigus, bullous Pemphigus, Epidermolysis bullosa, urticaria, angiodermas, vasculitides, erythemas, cutaneous eosinophilias, uveitis, Alopecia areata and vernal conjunctivitis;
(4) (**gastrointestinal tract**) Coeliac disease, proctitis, eosinopilic gastro-enteritis, mastocytosis, Crohn's disease, ulcerative colitis, food-related allergies which have effects remote from the gut, e.g., migraine, rhinitis and eczema;
(5) (**other tissues and systemic disease**) multiple sclerosis, atherosclerosis, Acquired Immunodeficiency Syndrome (AIDS), lupus erythematosus, systemic lupus, erythematosus, Hashimoto's thyroiditis, myasthenia gravis, type I diabetes, nephrotic syndrome, eosinophilia fascitis, hyper IgE syndrome, lepromatous leprosy, sezary syndrome and idiopathic thrombocytopenia pupura;
(6) (**allograft rejection**) acute and chronic following, for example, transplantation of kidney, heart, liver, lung, bone marrow, skin and cornea; and chronic graft versus host disease;
(7) cancers, especially non-small cell lung cancer (NSCLC) and squamous sarcoma;
(8) diseases in which angiogenesis is associated with raised chemokine levels (e.g. NSCLC); and
(9) cystic fibrosis, stroke, re-perfusion injury in the heart, brain, peripheral limbs and sepsis.

Thus, the present invention provides a compound of formula (I), or a pharmaceutically-acceptable salt or solvate thereof, as hereinbefore defined for use in therapy.

In a further aspect, the present invention provides the use of a compound of formula (I), or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The terms "therapeutic" and "therapeutically" should be construed accordingly.

For the above-mentioned therapeutic uses the dosage administered will, of course, vary with the compound employed, the mode of administration, the treatment desired and the disorder indicated. The daily dosage of the compound of formula (I) may be in the range from 0.001 mg/kg to 30 mg/kg.

The compounds of formula (I) and pharmaceutically acceptable salts and solvates thereof may be used on their own but will generally be administered in the form of a pharmaceutical composition in which the formula (I) compound/salt/solvate (active ingredient) is in association with a pharmaceutically acceptable adjuvant, diluent or carrier. Depending on the mode of administration, the pharmaceutical composition will preferably comprise from 0.05 to 99 %w (per cent by weight), more preferably from 0.05 to 80 %w, still more preferably from 0.10 to 70 %w, and even more preferably from 0.10 to 50 %w, of active ingredient, all percentages by weight being based on total composition.

The present invention also provides a pharmaceutical composition comprising a compound of formula (I), or a pharmaceutically acceptable salt or solvate thereof, as hereinbefore defined, in association with a pharmaceutically acceptable adjuvant, diluent or carrier.

The invention further provides a process for the preparation of a pharmaceutical composition of the invention which comprises mixing a compound of formula (I), or a pharmaceutically acceptable salt or solvate thereof, as hereinbefore defined, with a pharmaceutically acceptable adjuvant, diluent or carrier.

The pharmaceutical compositions may be administered topically (e.g. to the lung and/or airways or to the skin) in the form of solutions, suspensions, heptafluoroalkane aerosols and dry powder formulations; or systemically, e.g. by oral administration in the form of tablets, capsules, syrups, powders or granules, or by parenteral administration in the form of solutions or suspensions, or by subcutaneous administration or by rectal administration in the form of suppositories or transdermally.

The present invention will now be further explained by reference to the following illustrative Example.

### Example 1

### 4-({(2S)-3-[2-(acetylamino)-5-hydroxyphenoxy]-2-hydroxy-2-methylpropyl}ammonio)-1-(4-chlorobenzyl)piperidinium di(2,2,2-trifluoroacetate)

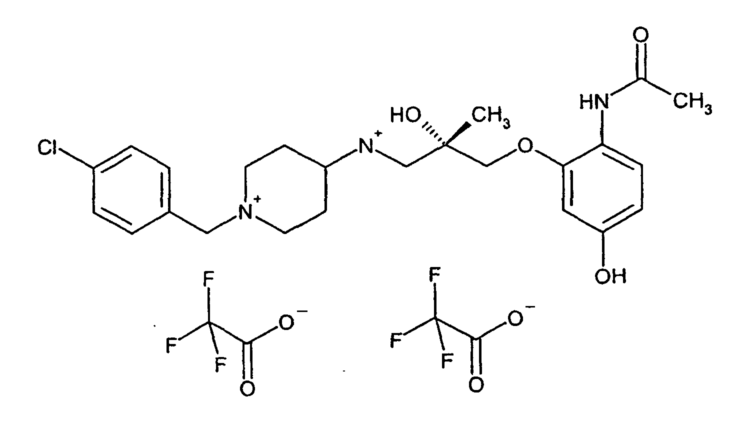

### i) N-(4-Methoxy-2-{[(2S)-2-methyloxiranyl]methoxy}phenyl)acetamide

A suspension of *N*-(2-hydroxy-4-methoxyphenyl)acetamide (1.04 g, 5.74 mmol), (2S)-2-[(3-nitrophenoxy)methyl]oxirane 2.04 g, 7.46 mmol) and cesium carbonate (2.80 g, 8.61 mmol) in dry dimethyl formamide (12.5 mL) was stirred at room temperature for 5 hours and then partitioned between ethyl-acetate and water. After extraction the combined organic phases were dried and concentrated *in vacuo.* The residue was purified by flash chromatography on silica, eluting with heptane:ethyl acetate, 1:1, to give the subtitled compound (1.19 g, 82.6% yield).

APCI-MS: m/z 252 (MH⁺).
¹H-NMR (CDCl₃, 400 MHz): δ 8.18 (d, 1H, J=8.8 Hz), 7.72 (bs, 1H), 6.52 (dd, 1H, J=2.6 and 8.8 Hz), 6.50 (dd, 1H, J=2.6 and 6.0 Hz), 4.10 (d, 1H, J=q11.0 Hz), 3.96 (d, 1H, J=11.0 Hz), 3.78 (s, 3H), 2.92 (d, 1H, J=4.6 Hz), 2.78 (d, 1H, J=4.6 Hz), 2.19 (s, 3H), 1.48 (s, 3H).

### ii) 4-({(2S)-3-[2-(acetylamino)-5-methoxyphenoxy]-2-hydroxy-2-methylpropyl}ammonio)-1-(4-chlorobenzyl)piperidinium di(2,2,2-trifluoroacetate)

A mixture of *N*-(4-methoxy-2-{[(2S)-2-methyloxiranyl]methoxy}phenyl)acetamide (182 mg, 0.72 mmol) and 1-(4-chlorobenzyl)-4-piperidinamine (163 mg, 0.72mmol) in ethanol (7.5 ml) was stirred at 80°C for 8 hours, then at room temperature overnight. The reaction mixture was concentrated and purified by reversed phase HPLC to give the sub-titled compound as a ditrifluoroacetate after lyophilisation (377mg, 74.4% yield).

APCI-MS: m/z 476 (MH⁺) for the free base.
¹H-NMR (CD₃OD, 400 MHz): δ 7.49 (m, 4H), 7.26 (d, 1H, J=8.6 Hz), 6.63 (d, 1H, J=2.4 Hz), 6.57 (dd, 1H, J=2.4 and 8.6 Hz), 4.3 (s, 2H), 3.95 (q, 2H, J=9.6 Hz), 3.79 (s, 3H), 3.59 (d, 2H, J=12.8 Hz), 3.44 (m, 1H), 3.10 (m, 3H), 2.40 (d, 2H, J=13.4 Hz), 2.12 (s, 3H), 2.04 (m, 2H), 1.40 (s, 3H).

### iii) 4-({(2S)-3-[2-(acetylamino)-5-hydroxyphenoxy]-2-hydroxy-2-methylpropyl}ammonio)-1-(4-chlorobenzyl)piperidinium di(2,2,2-trifluoroacetate)

4-({(2*S*)-3-[2-(acetylamino)-5-methoxyphenoxy]-2-hydroxy-2-methylpropyl}ammonio)-1-(4-chlorobenzyl)piperidinium di(2,2,2-trifluoroacetate) (185 mg, 0.26 mmol) was partitioned between ethyl acetate and 1M aqueous sodium hydroxide. The aqueous phase was extracted with ethyl acetate and the combined organic phase was dried and concentrated. The cold (0°C), stirred solution of residual free base in dry dichloromethane (15 mL) was treated with 1M boron tribromide in dichloromethane (1.58 mmol). The mixture was allowed to attain room temperature overnight. Methanol was added to obtain a clear solution and the mixture was concentrated. The crude product was purified by HPLC to afford, after freeze-drying, the titled compound (86 mg, 48%).

APCI-MS: m/z 462 (MH⁺) for the free base.
¹H-NMR (CD₃OD, 400 MHz): δ 7.49 (m, 4H), 7.09 (d, 1H, J=8.4 Hz), 6.51 (d, 1H, J=2.4 Hz), 6.41 (dd, 1H, J=2.4 and 8.4 Hz), 4.30 (s, 2H), 3.90 (dd, 2H, J=9.6 and 13.4 Hz), 3.37-3.64 (m, 4H), 3.10 (m, 3H), 2.39 (bd, 2H, J=13.1 H), 2.11 (s, 3H), 2.03 (m, 2H), 1.39 (s, 3H).

### THP-1 Chemotaxis Assay

### Introduction

The assay measured the chemotactic response elicited by MIP-1α chemokine in the human monocytic cell line THP-1. The compound of the Example was evaluated by its ability to depress the chemotactic response to a standard concentration of MIP-1α chemokine.

### Methods

### Culture of THP-1 cells

Cells were thawed rapidly at 37°C from frozen aliquots and resuspended in a 25 cm flask containing 5 ml of RPMI-1640 medium supplemented with Glutamax and 10% heat inactivated fetal calf serum without antibiotics (RPMI+10%HIFCS). At day 3 the medium is discarded and replaced with fresh medium.

THP-1 cells are routinely cultured in RPMI-1640 medium supplemented with 10% heat inactivated fetal calf serum and glutamax but without antibiotics. Optimal growth of the cells requires that they are passaged every 3 days and that the minimum subculture density is 4x10⁵ cells/ml.

### Chemotaxis assay

Cells were removed from the flask and washed by centrifugation in RPMI+10%HIFCS+glutamax. The cells were then resuspended at 2x10⁷ cells/ml in fresh medium (RPMI+10%HIFCS+glutamax) to which was added calcein-AM (5 µl of stock solution to 1 ml to give a final concentration of 5x10⁻⁶M). After gentle mixing the cells were incubated at 37°C in a CO₂ incubator for 30 minutes. The cells were then diluted to 50 ml with medium and washed twice by centrifugation at 400xg. Labelled cells were then resuspended at a cell concentration of 1x10 cells/ml and incubated with an equal volume of MIP-1α antagonist (10⁻¹⁰M to 10⁻⁶M final concentration) for 30 minutes at 37°C in a humidified CO₂ incubator.

Chemotaxis was performed using Neuroprobe 96-well chemotaxis plates employing 8 µm filters (cat no. 101-8). Thirty microlitres of chemoattractant supplemented with various concentrations of antagonists or vehicle were added to the lower wells of the plate in triplicate. The filter was then carefully positioned on top and then 25µl of cells preincubated with the corresponding concentration of antagonist or vehicle were added to the surface of the filter. The plate was then incubated for 2 hours at 37°C in a humidified CO₂ incubator. The cells remaining on the surface were then removed by adsorption and the whole plate was centrifuged at 2000 rpm for 10 minutes. The filter was then removed and the cells that had migrated to the lower wells were quantified by the fluorescence of cell associated calcein-AM. Cell migration was then expressed in fluorescence units after subtraction of the reagent blank and values were standardized to % migration by comparing the fluorescence values with that of a known number of labelled cells. The effect of antagonists was calculated as % inhibition when the number of migrated cells were compared with vehicle.

### hERGencoded Potassium Channel Inhibition Test

This assay determines the ability of a test compound to inhibit the tail current flowing through the human ether-a-go-go-related-gene (hERG)-encoded potassium channel.

Human embryonic kidney (HEK) cells expressing the hERG-encoded channel were grown in Minimum Essential Medium Eagle (EMEM; Sigma-Aldrich catalogue number M2279), supplemented with 10% Foetal Calf Serum (Labtech International; product number 4-101-500), 10% M1 serum-free supplement (Egg Technologies; product number 70916) and 0.4 mg/ml Geneticin G418 (Sigma-Aldrich; catalogue number G7034). One or two days before each experiment, the cells were detached from the tissue culture flasks with Accutase (TCS Biologicals) using standard tissue culture methods. They were then put onto glass coverslips resting in wells of a 12 well plate and covered with 2 ml of the growing media.

For each cell recorded, a glass coverslip containing the cells was placed at the bottom of a Perspex chamber containing bath solution (see below) at room temperature (~20 °C). This chamber was fixed to the stage of an inverted, phase-contrast microscope. Immediately after placing the coverslip in the chamber, bath solution was perfused into the chamber from a gravity-fed reservoir for 2 minutes at a rate of ~ 2 ml/min. After this time, perfusion was stopped.

A patch pipette made from borosilicate glass tubing (GC120F, Harvard Apparatus) using a P-97 micropipette puller (Sutter Instrument Co.) was filled with pipette solution (see hereinafter). The pipette was connected to the headstage of the patch clamp amplifier (Axopatch 200B, Axon Instruments) via a silver/silver chloride wire. The headstage ground was connected to the earth electrode. This consisted of a silver/silver chloride wire embedded in 3% agar made up with 0.85% sodium chloride.

The cell was recorded in the whole cell configuration of the patch clamp technique. Following "break-in", which was done at a holding potential of -80 mV (set by the amplifier), and appropriate adjustment of series resistance and capacitance controls, electrophysiology software *(Clampex,* Axon Instruments) was used to set a holding potential (-80 mV) and to deliver a voltage protocol. This protocol was applied every 15 seconds and consisted of a 1 s step to +40 mV followed by a I s step to -50 mV. The current response to each imposed voltage protocol was low pass filtered by the amplifier at 1 kHz. The filtered signal was then acquired, on line, by digitising this analogue signal from the amplifier with an analogue to digital converter. The digitised signal was then captured on a computer running *Clampex* software (Axon Instruments). During the holding potential and the step to + 40 mV the current was sampled at 1 kHz. The sampling rate was then set to 5 kHz for the remainder of the voltage protocol.

The compositions, pH and osmolarity of the bath and pipette solution are tabulated below.

| **Salt** | **Pipette Solution (mM)** | **Bath Solution (mM)** |
|---|---|---|
| NaCl | - | 137 |
| KCl | 130 | 4 |
| MgCl₂ | 1 | 1 |
| CaCl₂ | - | 1.8 |
| HEPES | 10 | 10 |
| glucose | - | 10 |
| Na₂ATP | 5 | - |
| EGTA | 5 | - |

| **Parameter** | **Pipette** | **Bath** |
|---|---|---|
| pH | 7.18 - 7.22 | 7.40 |
| pH adjustment with | 1M KOH | 1M NaOH |
| Osmolarity (mOsm) | 275-285 | 285-295 |

The amplitude of the hERG-encoded potassium channel tail current following the step from +40 mV to -50 mV was recorded on-line by *Clampex* software (Axon Instruments). Following stabilisation of the tail current amplitude, bath solution containing the vehicle for the test substance was applied to the cell. Providing the vehicle application had no significant effect on tail current amplitude, a cumulative concentration effect curve to the compound was then constructed.

The effect of each concentration of test compound was quantified by expressing the tail current amplitude in the presence of a given concentration of test compound as a percentage of that in the presence of vehicle.

Test compound potency (IC₅₀) was determined by fitting the percentage inhibition values making up the concentration-effect to a four parameter Hill equation using a standard data-fitting package. If the level of inhibition seen at the highest test concentration did not exceed 50%, no potency value was produced and a percentage inhibition value at that concentration was quoted.

The following table shows the results that were obtained when the compound of Example 1 above and three comparison compounds A, B and C were tested in the hERG-encoded potassium channel inhibition test:

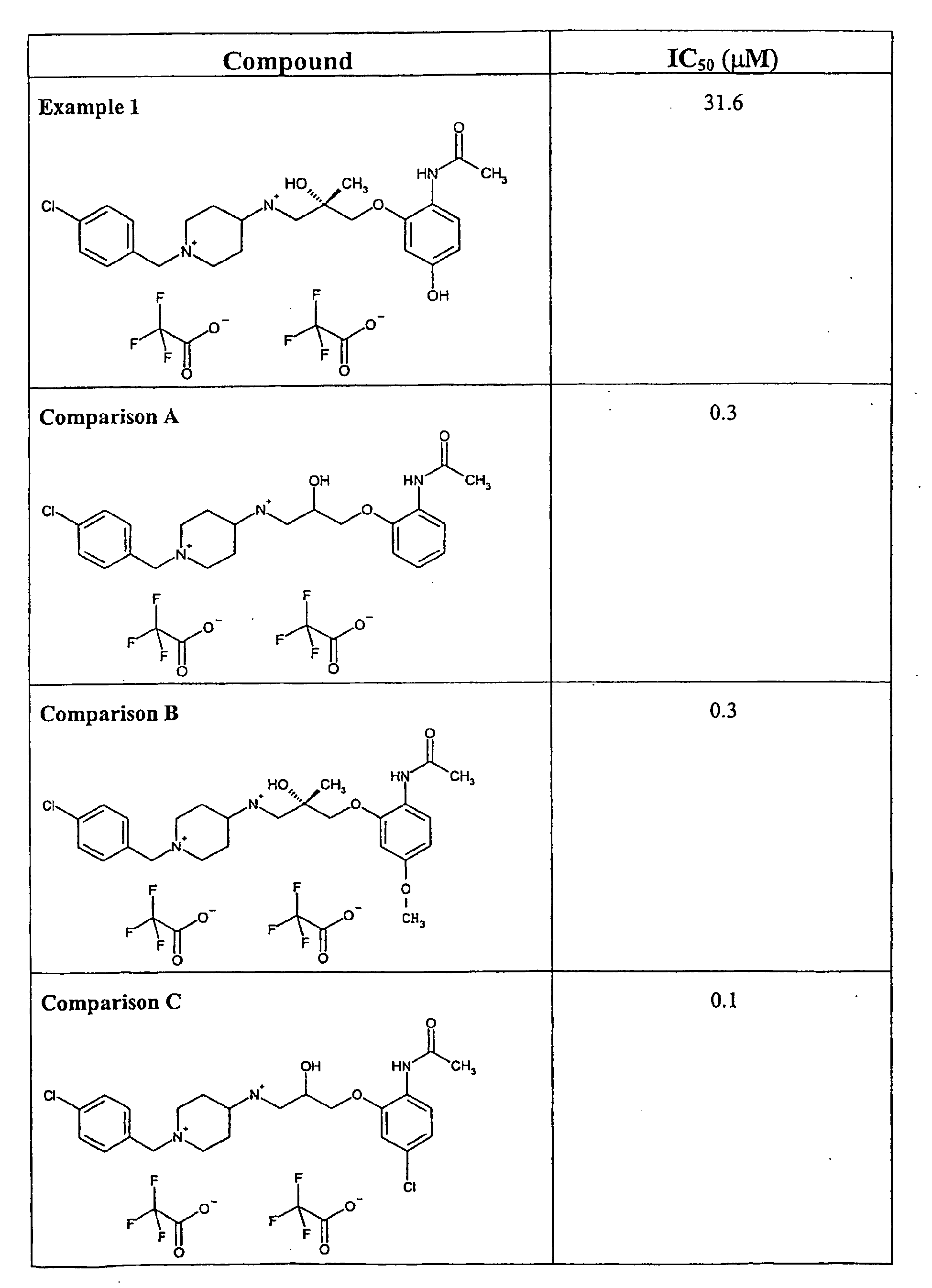

## Claims

1. A compound of formula 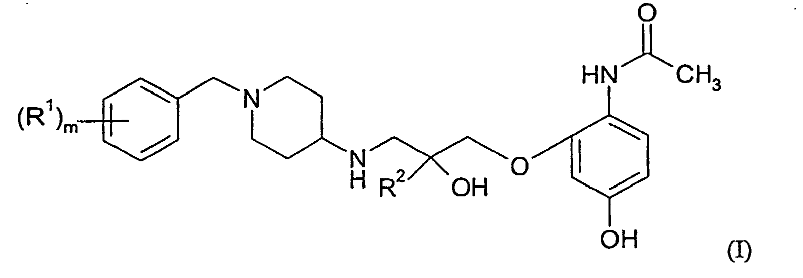 wherein
m is 0, 1 or 2;
each R¹ independently represents halogen or cyano; and
R² represents a hydrogen atom or methyl group;
or a pharmaceutically acceptable salt or solvate thereof.

2. A compound according to claim 1, wherein m is 1 and R¹ represents a halogen atom.

3. A compound according to claim 1 or claim 2, wherein m is 1 and R¹ represents a chlorine atom.

4. A compound according to any one of the preceding claims, wherein R² represents a methyl group.

5. A compound according to claim 1 being:
4-({(2*S*)-3-[2-(acetylamino)-5-hydroxyphenoxy]-2-hydroxy-2-methylpropyl)ammonio)-1-(4-chlorobenzyl)piperidine,
or a pharmaceutically acceptable salt or solvate thereof.

6. A compound according to claim 5, wherein the pharmaceutically acceptable salt is a salt selected from hydrochloride, trifluoroacetate, hydrobromide, phosphate, acetate, fumarate, maleate, tartrate, citrate, oxalate, methanesulphonate and p-toluenesulphonate.

7. A compound according to claim 5 or 6, wherein the pharmaceutically acceptable salt is a ditrifluoroacetate salt.

8. A process for preparing a compound of formula (I) or a pharmaceutically acceptable salt or solvate thereof as defined in claim 1 which comprises
(a) reacting a compound of formula 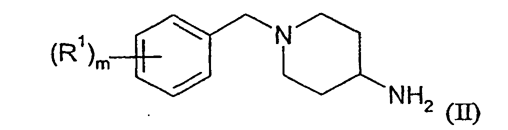 wherein m and R¹ are as defined in formula (I), with a compound of formula 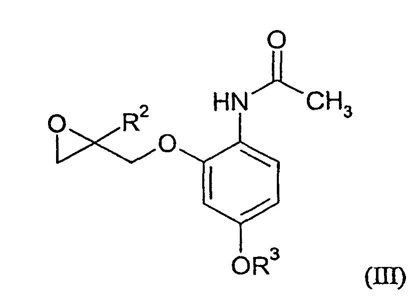 wherein R³ represents a hydrogen atom or a suitable protecting group and R² is as defined in formula (I); or
(b) reacting a compound of formula 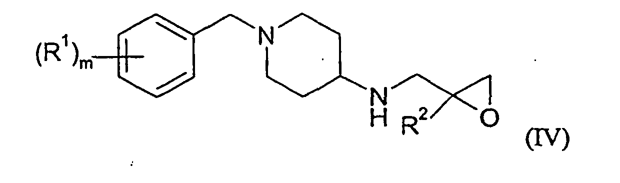 wherein m, R¹ and R² are as defined in formula (I), with a compound of formula 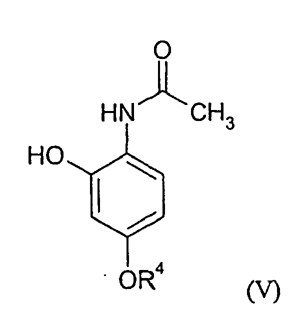 wherein R⁴ represents a hydrogen atom or a suitable protecting group; or
(c) reacting a compound of formula 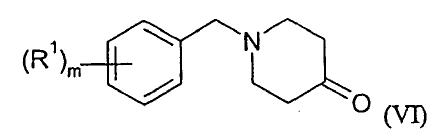 wherein m and R¹ are as defined in formula (I), with a compound of formula 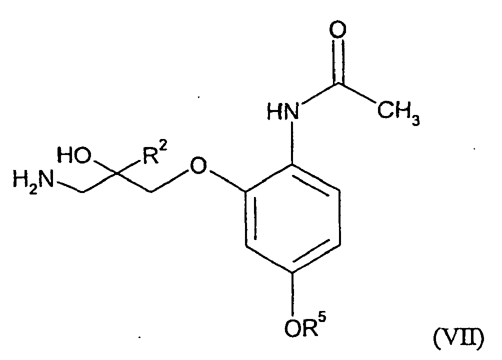 wherein R⁵ represents a hydrogen atom or a suitable protecting group and R² is as defined in formula (I);
and optionally after (a), (b) or (c) forming a pharmaceutically acceptable salt or solvate of the compound of formula (I).

9. A pharmaceutical composition comprising a compound of formula (I), or a pharmaceutically acceptable salt or solvate thereof, as claimed in any one of claims 1 to 7 in association with a pharmaceutically acceptable adjuvant, diluent or carrier.

10. A process for the preparation of a pharmaceutical composition as claimed in claim 9 which comprises mixing a compound of formula (I), or a pharmaceutically acceptable salt or solvate thereof, as claimed in any one of claims 1 to 7 with a pharmaceutically acceptable adjuvant, diluent or carrier.

11. A compound of formula (I), or a pharmaceutically acceptable salt or solvate thereof, as claimed in any one of claims 1 to 7 for use in therapy.

12. Use of a compound of formula (I), or a pharmaceutically acceptable salt or solvate thereof, as claimed in any one of claims 1 to 7 in the manufacture of a medicament for the treatment of human diseases or conditions in which modulation of chemokine receptor activity is beneficial.

13. Use of a compound of formula (I), or a pharmaceutically acceptable salt or solvate thereof, as claimed in any one of claims 1 to 7 in the manufacture of a medicament for use in treating rheumatoid arthritis.

14. Use of a compound of formula (I), or a pharmaceutically acceptable salt or solvate thereof, as claimed in any one of claims 1 to 7 in the manufacture of a medicament for use in treating chronic obstructive pulmonary disease.

15. Use of a compound of formula (I), or a pharmaceutically acceptable salt or solvate thereof, as claimed in any one of claims 1 to 7 in the manufacture of a medicament for use in treating asthma.

16. Use of a compound of formula (I), or a pharmaceutically acceptable salt or solvate thereof, as claimed in any one of claims 1 to 7 in the manufacture of a medicament for use in treating multiple sclerosis.

## Patentansprüche

1. Verbindungen der allgemeinen Formel 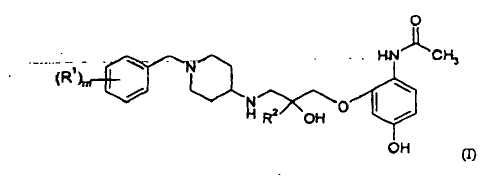 wobei:
m für 0, 1 oder 2 steht;
die Reste R¹ jeweils unabhängig voneinander für Halogen oder Cyano stehen; und
R² für ein Wasserstoffatom oder eine Methylgruppe steht;
und deren pharmazeutisch annehmbare Salze und Solvate.

2. Verbindungen nach Anspruch 1, wobei m für 1 steht und R¹ für ein Halogenatom steht.

3. Verbindungen nach Anspruch 1 oder 2, wobei m für 1 steht und R¹ für ein Chloratom steht.

4. Verbindungen nach einem der vorhergehenden Ansprüche, wobei R² für eine Methylgruppe steht.

5. Verbindung nach Anspruch 1, bei der es sich um:
4-({(2S)-3-[2-(Acetylamino)-5-hydroxyphenoxyl-2-hydroxy-2-methylpropyllammonio)-1-(4-chlorbenzyl)piperidin
handelt,
und deren pharmazeutisch annehmbare Salze und Solvate.

6. Verbindung nach Anspruch 5, wobei es sich bei dem pharmazeutisch annehmbaren Salz um ein Salz ausgewählt aus Hydrochlorid, Trifluoracetat, Hydrobromid, Phosphat, Acetat, Fumarat, Maleat, Tartrat, Citrat, Oxalat, Methansulfonat und p-Toluolsulfonat handelt.

7. Verbindung nach Anspruch 5 oder 6, wobei es sich bei dem pharmazeutisch annehmbaren Salz um ein Ditrifluoracetatsalz handelt.

8. Verfahren zur Herstellung einer wie in Anspruch 1 definierten Verbindung der Formel (I) bzw. einem pharmazeutisch annehmbaren Salz oder Solvat davon, bei dem man
(a) eine Verbindung der allgemeinen Formel 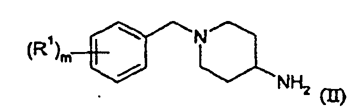 wobei m und R¹ wie in Formel (I) definiert sind, mit einer Verbindung der allgemeinen Formel 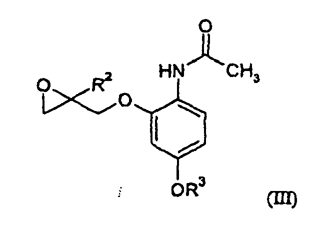 wobei R³ für ein Wasserstoffatom oder eine geeignete Schutzgruppe steht und R² wie in Formel (I) definiert ist, umsetzt; oder
(b) eine Verbindung der allgemeinen Formel 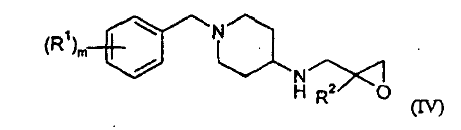 wobei m, R¹ und R² wie in Formel (I) definiert sind, mit einer Verbindung der allgemeinen Formel 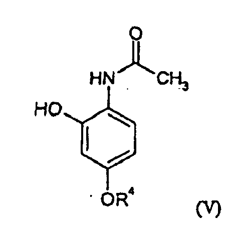 wobei R⁴ für ein Wasserstoffatom oder eine geeignete Schutzgruppe steht, umsetzt; oder
(c) eine Verbindung der allgemeinen Formel 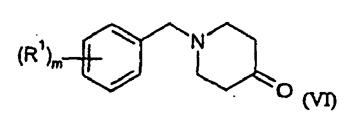 wobei m und R¹ wie in Formel (I) definiert sind, mit einer Verbindung der allgemeinen Formel 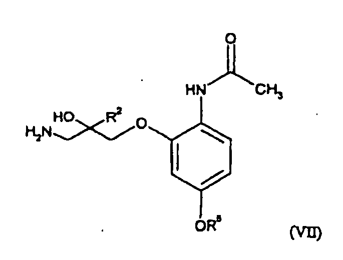 wobei R⁵ für ein Wasserstoffatom oder eine geeignete Schutzgruppe steht und R² wie in Formel (I) definiert ist, umsetzt;
und anschließend gegebenenfalls nach (a), (b) bzw. (c) ein pharmazeutisch annehmbares Salz oder Solvat der Verbindung der Formel (I) bildet.

9. Pharmazeutische Zusammensetzung, enthaltend eine Verbindung der Formel (I) oder ein pharmazeutisch annehmbares Salz oder Solvat davon nach einem der Ansprüche 1 bis 7 zusammen mit einem pharmazeutisch annehmbaren Adjuvans, Verdünnungsmittel oder Träger.

10. Verfahren zur Herstellung einer pharmazeutischen Zusammensetzung nach Anspruch 9, bei dem man eine Verbindung der Formel (I) oder ein pharmazeutisch annehmbares Salz oder Solvat davon nach einem der Ansprüche 1 bis 7 mit einem pharmazeutisch annehmbaren Adjuvans, Verdünnungsmittel oder Träger mischt.

11. Verbindungen der Formel (I) und deren pharmazeutisch annehmbare Salze und Solvate nach einem der Ansprüche 1 bis 7 zur Verwendung in der Therapie.

12. Verwendung einer Verbindung der Formel (I) oder eines pharmazeutisch annehmbaren Salzes oder Solvates davon nach einem der Ansprüche 1 bis 7 bei der Herstellung eines Medikaments zur Behandlung von menschlichen Erkrankungen oder Leiden, bei denen die Modulierung der Chemokinrezeptoraktivität von Nutzen ist.

13. Verwendung einer Verbindung der Formel (I) oder eines pharmazeutisch annehmbaren Salzes oder Solvates davon nach einem der Ansprüche 1 bis 7 bei der Herstellung eines Medikaments zur Verwendung bei der Behandlung von rheumatoider Arthritis.

14. Verwendung einer Verbindung der Formel (I) oder eines pharmazeutisch annehmbaren Salzes oder Solvates davon nach einem der Ansprüche 1 bis 7 bei der Herstellung eines Medikaments zur Verwendung bei der Behandlung von chronischobstruktiver Atemwegserkrankung.

15. Verwendung einer Verbindung der Formel (I) oder eines pharmazeutisch annehmbaren Salzes oder Solvates davon nach einem der Ansprüche 1 bis 7 bei der Herstellung eines Medikaments zur Verwendung bei der Behandlung von Asthma.

16. Verwendung einer Verbindung der Formel (I) oder eines pharmazeutisch annehmbaren Salzes oder Solvates davon nach einem der Ansprüche 1 bis 7 bei der Herstellung eines Medikaments zur Verwendung bei der Behandlung von multipler Sklerose.

## Revendications

1. Composé de formule 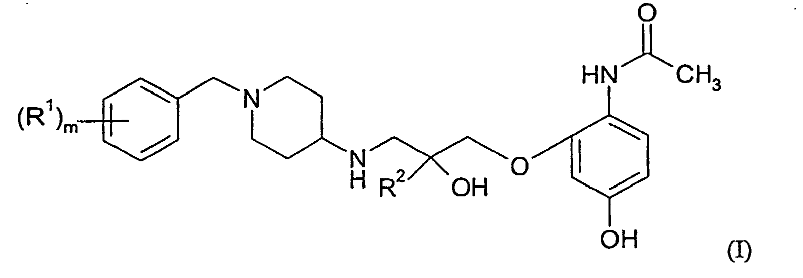 dans laquelle
m vaut 0, 1 ou 2 ;
chaque R¹ représente indépendamment un halogène ou un cyano ; et
R² représente un atome d'hydrogène ou un groupe méthyle ;
ou un sel ou solvate pharmaceutiquement acceptable de celui-ci.

2. Composé selon la revendication 1, dans lequel m vaut 1 et R¹ représente un atome d'halogène.

3. Composé selon la revendication 1 ou la revendication 2, dans lequel m vaut 1 et R¹ représente un atome de chlore.

4. Composé selon l'une quelconque des revendications précédentes, dans lequel R² représente un groupe méthyle.

5. Composé selon la revendication 1, à savoir :
la 4-({(2S)-3-[2-(acétylamino)-5-hydroxyphénoxy]-2-hydroxy-2-méthylpropyl]ammonio)-1-(9-chlorobenzyl)pipéridine,
ou un sel ou solvate pharmaceutiquement acceptable de celle-ci.

6. Composé selon la revendication 5, dans lequel le sel pharmaceutiquement acceptable est un sel choisi parmi un chlorhydrate, un trifluoroacétate, un bromhydrate, un phosphate, un acétate, un fumarate, un maléate, un tartrate, un citrate, un oxalate, un méthanesulfonate, un p-toluènesulfonate.

7. Composé selon la revendication 5 ou 6, dans lequel le sel pharmaceutiquement acceptable est un sel de ditrifluoroacétate.

8. Procédé de préparation d'un composé de formule (I) ou d'un sel ou solvate pharmaceutiquement acceptable de celui-ci tel que défini dans la revendication 1, comprenant
(a) la réaction d'un composé de formule 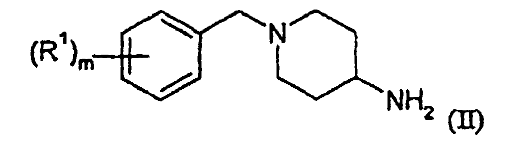 dans laquelle m et R¹ sont tels que définis dans la formule (I), avec un composé de formule 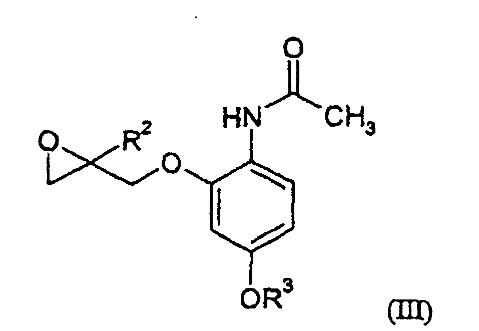 dans laquelle R³ représente un atome d'hydrogène ou un groupe protecteur approprié et R² est tel que défini dans la formule (I) ; ou
(b) la réaction d'un composé de formule 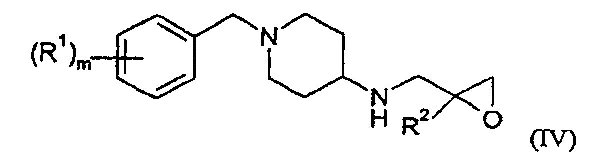 dans laquelle m, R¹ et R² sont tels que définis dans la formule (I), avec un composé de formule 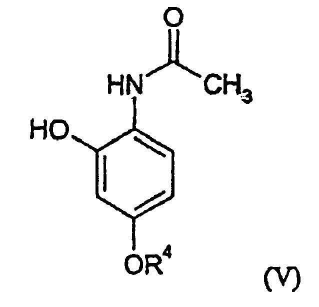 dans laquelle R⁴ représente un atome d'hydrogène ou un groupe protecteur approprié ; ou
(c) la réaction d'un composé de formule 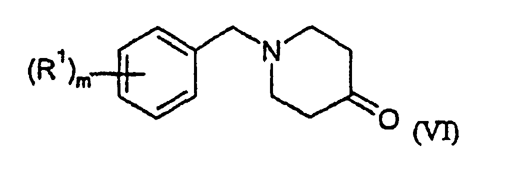 dans laquelle m et R¹ sont tels que définis dans la formule (I), avec un composé de formule 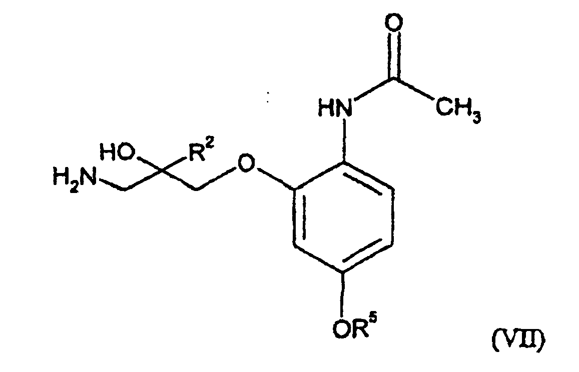 dans laquelle R⁵ représente un atome d'hydrogène ou un groupe protecteur approprié et R² est tel que défini dans la formule (I) ;
et éventuellement après (a), (b) ou (c), la formation d'un sel ou solvate pharmaceutiquement acceptable du composé de formule (I).

9. Composition pharmaceutique comprenant un composé de formule (I), ou un sel ou solvate pharmaceutiquement acceptable de celui-ci, selon l'une quelconque des revendications 1 à 7, en association avec un adjuvant, un diluant ou un support pharmaceutiquement acceptable.

10. Procédé de préparation d'une composition pharmaceutique selon la revendication 9, comprenant la mixtion d'un composé de formule (I) ou d'un sel ou solvate pharmaceutiquement acceptable de celui-ci, selon l'une quelconque des revendications 1 à 7, avec un adjuvant, un diluant ou un support pharmaceutiquement acceptable.

11. Composé de formule (I), ou sel ou solvate pharmaceutiquement acceptable de celui-ci, selon l'une quelconque des revendications 1 à 7, destiné à être utilisé en thérapie.

12. Utilisation d'un composé de formule (I), ou d'un sel ou solvate pharmaceutiquement acceptable de celui-ci, selon l'une quelconque des revendications 1 à 7, pour la fabrication d'un médicament destiné au traitement de maladies ou de pathologies humaines pour lesquelles une modulation de l'activité des récepteurs de chimiokines est bénéfique.

13. Utilisation d'un composé de formule (I), ou d'un sel ou solvate pharmaceutiquement acceptable de celui-ci, selon l'une quelconque des revendications 1 à 7, pour la fabrication d'un médicament destiné à être utilisé pour le traitement de la polyarthrite rhumatoïde.

14. Utilisation d'un composé de formule (I), ou d'un sel ou solvate pharmaceutiquement acceptable de celui-ci, selon l'une quelconque des revendications 1 à 7, pour la fabrication d'un médicament destiné à être utilisé pour le traitement de la maladie pulmonaire obstructive chronique.

15. Utilisation d'un composé de formule (I), ou d'un sel ou solvate pharmaceutiquement acceptable de celui-ci, selon l'une quelconque des revendications 1 à 7, pour la fabrication d'un médicament destiné à être utilisé pour le traitement de l'asthme.

16. Utilisation d'un composé de formule (I), ou d'un sel ou solvate pharmaceutiquement acceptable de celui-ci, selon l'une quelconque des revendications 1 à 7, pour la fabrication d'un médicament destiné à être utilisé pour le traitement de la sclérose en plaques.
